(19) Europäisches Patentamt / European Patent Office / Office européen des brevets

(11) EP 2 626 073 A1

(12) **EUROPEAN PATENT APPLICATION**

(43) Date of publication:
**14.08.2013 Bulletin 2013/33**

(21) Application number: **12181152.5**

(22) Date of filing: **21.08.2012**

(51) Int Cl.:
*A61K 31/4709* (2006.01) *A61P 25/28* (2006.01)
*A61P 9/10* (2006.01) *A61P 17/06* (2006.01)
*A61P 29/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.02.2012 US 201261598139 P**

(71) Applicants:
- **Harmonic Pharma**
  **54600 Villers-les-Nancy (FR)**
- **Synaging**
  **54000 Nancy (FR)**

(72) Inventors:
- **Souchet, Michel**
  **91190 Gif-sur-Yvette (FR)**
- **Karaboga, Arnaud Sinan**
  **67100 Strasbourg (FR)**
- **Pillot, Thierry**
  **54110 Crévic (FR)**

(74) Representative: **Icosa**
**83 avenue Denfert-Rochereau**
**75014 Paris (FR)**

(54) **Compound for use in the prevention and/or treatment of a neurogenerative disease or a disease involving an activation of phosphodiesterase-4 (PDE4)**

(57) The present invention relates to a compound for use in the prevention and/or treatment of a disease selected from a neurodegenerative disease, especially Alzheimer disease, and a disease involving an activation of phosphodiesterase-4 (PDE4), wherein said compound is of general formula (I) or a pharmaceutically acceptable salt or solvate thereof:

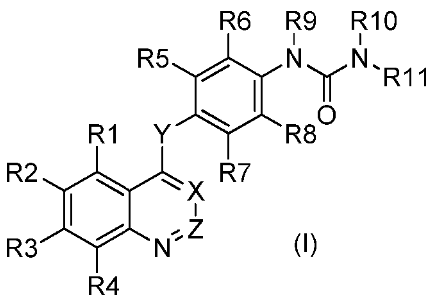

(I)

wherein X and Z each independently represent CH or N; Y represents O or S;
R1, R2 and R3 may be the same or different and represent a hydrogen atom, a halogen atom, hydroxyl, nitro, cyano, amino, amido, carbamate, alkyl, alkenyl, alkynyl, alkoxy, heterocyclic, carbocyclic, alkylthio, carboxy, or alkoxycarbonyl; R4 represent a hydrogen atom; R5, R6, R7 and R8 may be the same or different and represent a hydrogen atom, halogen atom, hydroxyl, nitro, amino, alkyl, alkoxy, alkylthio, or trifluoromethyl; R9 and R10 may be the same or different and represent a hydrogen atom, alkyl, alkylcarbonyl, heterocyclic, carbocyclic, $ONO_2$, or $OSO_2R$ wherein R is hydroxyl, alkyl, carbocyclic; and R11 represents an alkyl, alkenyl, alkynyl, alkoxy, akoxycarbonyl, amino, amido, heterocyclic, carbocyclic, or optionally substituted azolyl.



Printed by Jouve, 75001 PARIS (FR)

## Description

### FIELD OF INVENTION

The invention relates to the field of the prevention and/or treatment of diseases involving an activation of phosphodiesterase-4 (PDE4). Especially, the present invention relates to modulators of phosphodiesterase-4 (PDE4), preferably allosteric modulators of PDE4, for use in the prevention and/or treatment of a disease involving an activation of PDE4, such as for example a neurodegenerative disease. Preferably, said modulators of phosphodiesterase-4 (PDE4) have the following general formula (I):

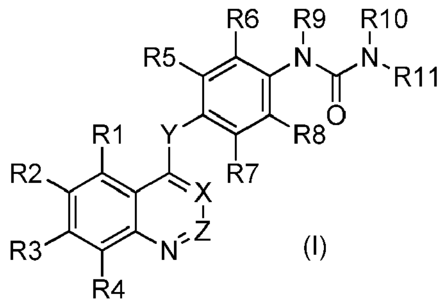

(I)

or a pharmaceutically acceptable salt or solvate thereof,
wherein X and Z each independently represent CH or N;
Y represents O or S;
R1, R2 and R3 may be the same or different and represent a hydrogen atom, a halogen atom, hydroxyl, nitro, cyano, amino, amido, carbamate, alkyl, alkenyl, alkynyl, alkoxy, heterocyclic, carbocyclic, alkylthio, carboxy, or alkoxycarbonyl;
R4 represent a hydrogen atom;
R5, R6, R7 and R8 may be the same or different and represent a hydrogen atom, halogen atom, hydroxyl, nitro, amino, alkyl, alkoxy, alkylthio, or trifluoromethyl;
R9 and R10 may be the same or different and represent a hydrogen atom, alkyl, alkylcarbonyl, heterocyclic, carbocyclic, $ONO_2$, or $OSO_2R$ wherein R is hydroxyl, alkyl, carbocyclic; and
R11 represents an alkyl, alkenyl, alkynyl, alkoxy, akoxycarbonyl, amino, amido, heterocyclic, carbocyclic, or optionally substituted azolyl.

Preferably, the disease involving an activation of PDE4 is a neurodegenerative disease such as for example Alzheimer disease, Parkinson disease, multiple sclerosis or amyotrophic lateral sclerosis.

### BACKGROUND OF INVENTION

Phosphodiesterase-4 (PDE4) is an enzyme belonging to the phosphodiesterase family. Phosphodiesterases are enzymes that break phosphodiester bonds. Especially, phosphodiesterase-4 (PDE4) regulates the intracellular level of cyclic adenosine (cAMP) by hydrolyzing cAMP within both immune cells and cells in the central nervous system (CNS). PDE4 inhibitors prevent the inactivation of the intracellular messenger cAMP.

PDE4 is therefore a therapeutic target of high interest for neurological diseases, neurodegenerative diseases, inflammatory diseases, respiratory diseases, allergies, cardiovascular diseases, thrombosis, diabetes, cancer, rheumatoid arthritis, atopic dermatitis, psoriasis, asthma, inflammatory bowel disease.

Neurodegenerative diseases are characterized by a progressive and irreversible deterioration of brain cells and eventually death of the neurons. Neurodegenerative diseases referred to in the present invention may or may not be associated with neuroinflammatory processes or to PDE4 activation processes. Neurodegenerative diseases comprise among others, but are not limited to, Alzheimer disease, Parkinson disease, multiple sclerosis or amyotrophic lateral sclerosis, and any disease wherein a progressive loss of structure or function of neurons, including death of neurons, occurs. Efficient treatments for preventing, stopping and/or reversing disease involving an activation of PDE4, especially neurodegenerative diseases are needed.

Indeed, no PDE4 inhibitors have yet been brought to market because of issues related to tolerability and side effects such as emesis and diarrhea. The emetic response to PDE4 inhibitors is mediated in part by a brainstem noradrenergic pathway and can be reduced by limiting distribution to the brain. However, when distribution to brain is desired for therapeutic benefit, as in central nervous system (CNS) indications such as impaired cognition, schizophrenia and depression, a different strategy is required.

The PDE4 inhibitors that have been explored in human clinical trials bind the active site competitively with

cAMP and therefore completely inhibit enzyme activity at high concentrations. Although this traditional approach to PDE4 inhibitor design has demonstrated therapeutic benefit, competitive inhibitors are likely to alter cAMP concentrations beyond normal physiological levels, perturbing the tight temporal and spatial control of cAMP signaling within cells, and leading to side effects.

As an alternative approach, some authors have designed PDE4 allosteric modulators that only partially inhibit cAMP hydrolysis. Such compounds are more likely to lower the magnitude of PDE4 inhibition and maintain cAMP signaling, thereby reducing target-based toxicity. A similar approach has been described previously for allosteric modulators of G protein-coupled receptors and for atypical retinoid ligands of nuclear hormone receptors.

Some authors showed that PDE4 allosteric modulators are potent in cellular and *in vivo* assays and have greatly reduced potential for emesis.

Therefore, this is an object of the present invention to provide further PDE4 allosteric modulators for the prevention and/or treatment of diseases involving an activation of PDE4, especially neurodegenerative diseases, said PDE4 allosteric modulators being efficient while not leading to side-effect such as emesis or diarrhea.

The identification of PDE4 allosteric modulators for the treatment of diseases involving an activation of PDE4 of the invention was performed by a unique *in silico* selection process developed by the Applicant. The activity of *in silico* selected compounds was then confirmed by *in vitro* and *in vivo* assays.

For the *in silico* selection process, the Applicant used its proprietary method of generating spherical harmonic (SH) based molecular representation of compounds of therapeutic interest. As represented on figure 1 for a cholesterol-like compound, small molecules may be converted into their spherical harmonic (SH) based molecular representation by successive deflation processes. This method was applied to known PDE4 allosteric modulators to determine a common SH shape. For example, the cognate SH shapes of compound D159687 was calculated as represented on figure 2. Compound D159687 was described as being a PDE4 allosteric modulators by Burgin et al. in Nature Biotechnology, Vol. 28, Number 1, January 2010, pp. 63-72.

Pharmacophoric based representations of PDE4 modulators were also established, as represented on figure 3 for compound D 159153.

The common characteristic SH shape and pharmacophoric representations of PDE4 allosteric modulators were then compared with a proprietary collection of approved or clinical drugs. This comparison gave rise to a set of hit compounds. The hit list was ranked according to a consensus score.

The best compounds of the *in silico* selection were retained for experimental *in vitro* and *in vivo* assessment for the treatment of diseases involving an activation of PDE4, leading to the selection of compounds described in the present invention. In particular, compounds of the invention were tested in neurodegenerative diseases preclinical models, especially in Alzheimer preclinical models.

The present invention thus relates to compounds of general formula (I) or a pharmaceutically acceptable salt or solvate of thereof, for use in the prevention and/or treatment of a neurodegenerative disease and/or a disease involving an activation of PDE4, said formula (I) being the following:

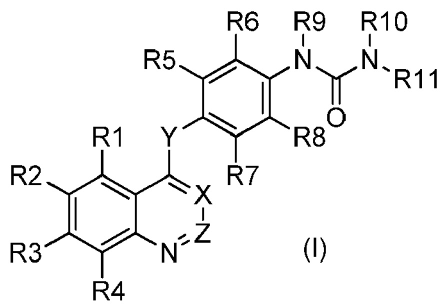

(I)

wherein X and Z each independently represent CH or N;
Y represents O or S;
R1, R2 and R3 may be the same or different and represent a hydrogen atom, a halogen atom, hydroxyl, nitro, cyano, amino, amido, carbamate, alkyl, alkenyl, alkynyl, alkoxy, heterocyclic, carbocyclic, alkylthio, carboxy, or alkoxycarbonyl;
R4 represent a hydrogen atom;
R5, R6, R7 and R8 may be the same or different and represent a hydrogen atom, halogen atom, hydroxyl, nitro, amino, alkyl, alkoxy, alkylthio, or trifluoromethyl;
R9 and R10 may be the same or different and represent a hydrogen atom, alkyl, alkylcarbonyl, heterocyclic, carbocyclic, $ONO_2$, or $OSO_2R$ wherein R is hydroxyl, alkyl, carbocyclic; and
R11 represents an alkyl, alkenyl, alkynyl, alkoxy, akoxycarbonyl, amino, amido, heterocyclic, carbocyclic, or optionally substituted azolyl.

In a preferred embodiment, the present invention relates to tivozanib or a pharmaceutically acceptable salt or solvate of thereof, for use in the prevention and/or treatment of a disease selected from a neurodegerative disease and a disease involving an activation of PDE4, preferably the treatment of Alzheimer disease, wherein so-called compound tizovanib (Av-951, KRN951), is of formula:

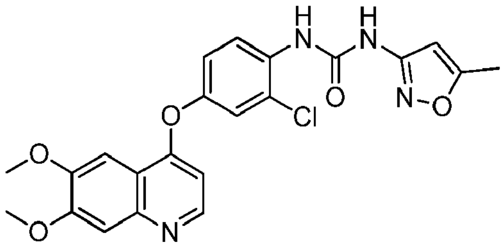

In another preferred embodiment, the present invention relates to 1-(2-chloro-4-((6,7-dimethoxyquinazolin-4-yl)oxy)phenyl)-3-propylurea or a pharmaceutically acceptable salt or solvate of thereof, for use in the prevention and/or treatment of a disease involving an activation of PDE4, of following formula:

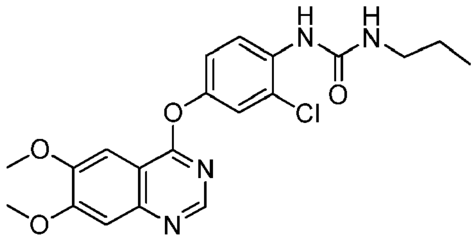

Compounds of the present invention were first described in patent applications EP1 382 604, EP 1447 405, EP 1652 847, EP 1 384 712, EP 1 535 910, EP 0 860 433, EP 1 153 920, and EP 1 566 379. Especially, compounds of the present invention were reported as antitumor agents.

To the knowledge of the Applicant, the use of the compounds of the present invention, and especially of tivozanib, as PDE4 allosteric modulators was never reported before.

Moreover, to the knowledge of the Applicant, the use of the compounds of the present invention, and especially of tivozanib, for the prevention and/or treatment of diseases such as neurodegenerative diseases and/or diseases involving an activation of PDE4 was never reported before.

**DEFINITIONS**

In the present invention, the following terms have the following meanings:
When applicable, groups described below may be substituted with one or more substituents, and preferably with one, two or three substituents. Substituents may be selected from but not limited to, for example, the group comprising halogen, hydroxyl, oxo, nitro, amido, carboxy, amino, cyano, haloalkoxy, haloalkyl, alkyl 1, alkenyl, alkynyl, carbocyclic group, heterocyclic group, carbocyclic group, azolyl, alkoxy, alkoxycarbonyl, alkylcarbonyl, alkylthio, trifluoromethyl, heterocycliccarbonyl, carbocycliccarbonyl.

- **"alkyl"** refers to any saturated linear or branched hydrocarbon chain, with 1 to 10, preferably 1 to 6 carbon atoms, more preferably 1 to 5 carbon atoms, and even more preferably 1 to 4 carbon atoms. The alkyl group may be substituted, such as for example by a substituted or unsubstituted aryl group. Suitable alkyl groups include methyl, ethyl, *n*-propyl, *iso*-propyl, *n*-butyl, *sec*-butyl, *iso*-butyl, *tert*-butyl, *n*-pentyl, *iso*-pentyl, *sec*-pentyl, *tert*-pentyl, *neo*-pentyl, *n*-hexyl, *sec*- hexyl, *iso*- hexyl, *tert*-hexyl, and *neo*-hexyl.

- **"alkenyl"** refers to any linear or branched hydrocarbon chain having at least one double bond, of 2 to 6 carbon atoms, preferably 2 to 5 carbon atoms, and more preferably 2 to 4 carbon atoms. The alkenyl group may be substituted, such as for example by a substituted or unsubstituted aryl group. Examples of alkenyl groups are ethenyl, 2- propenyl, 2-butenyl, 3-butenyl, 2-pentenyl and its isomers, 2-hexenyl and its isomers, 2,4-pentadienyl and the like.

- **"alkynyl"** refers to any linear or branched hydrocarbon chain having at least one triple bond, of 2 to 6 carbon atoms, and preferably 2 to 4 carbon atoms. The alkynyl group may be substituted. Non limiting examples of alkynyl groups

are ethynyl, 2-propynyl, 2-butynyl, 3-butynyl, 2-pentynyl and its isomers, 2-hexynyl and its isomers-and the like.

- **"carbocyclic"** refers to a substituted or not substituted carbocyclic substituent, that is to say, a monovalent, saturated, or unsaturated hydrocarbyl group having 1 or 2 cyclic structures. Carbocyclic group includes monocyclic or bicyclic hydrocarbyl groups. Carbocyclic groups may comprise 3 or more carbon atoms in the ring and generally, according to this invention comprise from 3 to 12, preferably 3 to 10, more preferably from 3 to 8 carbon atoms, still more preferably from 3 to 7 and even more preferably 3 to 6 carbon atoms. Examples of carbocyclic groups include but are not limited to cyclopropyl, cyclobutyl, cyclopentyl, cyclohexyl, cycloheptyl, phenyl, naphthyl.

- **"heteroatom"** refers to an oxygen, nitrogen, or sulfur atom.

- **"heterocyclic"** refers to a substituted or not substituted, saturated or unsaturated ring having 1 or 2 cyclic structures and containing one or more heteroatoms selected from oxygen, nitrogen, and sulfur atoms. Heterocyclic groups include monocyclic or bicyclic groups and may comprise 3 or more carbon atoms in the ring and generally, according to this invention comprise from 3 to 10, preferably from 3 to 8 carbon atoms, more preferably from 3 to 7, still more preferably 5 to 7 carbon atoms and even more preferably 5 or 6 carbon atoms. Examples of heterocyclic groups include but are not limited to pyridyl, pyrazinyl, pyrimidinyl, pyridazinyl, pyrrolidinyl, piperidinyl, piperazinyl, homopyperazinyl, morpholinyl, quinolyl, and quinazolinyl,

- **"azolyl"** refers to a five-membered saturated or unsaturated heterocyclic group containing two or more heteroatoms as ring atoms, selected in the group comprising nitrogen, sulfur and oxygen atoms, wherein at least one of the heteroatoms is a nitrogen atom, and on which one or more hydrogen atoms are optionally substituted by a halogen atom; C1-4 alkyl; C1-4 alkoxy; C1-4 alkylthio; trifluoromethyl; nitro; amino on which one or two hydrogen atoms are optionally substituted by C1-4 alkyl group(s) which may be the same or different; C1-4 alkoxycarbonyl, C1-4 alkyl, C1-4 alkylcarbonyl or C3-5 cyclic alkyl.

- **"halogen"** refers to fluorine, chlorine, bromine or iodine atom. Preferred halogen groups are fluorine and chlorine atoms.

- **"haloalkyl"** refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoro methyl, 1,1,1-trifluoroethyl and the like.

- **"haloalkoxy"** refers to an alkoxy radical having the meaning as defined above wherein one or more hydrogens are replaced with a halogen as defined above.

- **"alkoxy"** refers to any O-alkyl group. Non-limiting examples of such alkoxy are $C_{1-6}$ alkoxy selected in the group comprising methoxy, ethoxy, *n*-propoxy, *iso*propoxy, *n*-butoxy, *sec*-butoxy, *iso*-butoxy, *tert*-butoxy, *n*-pentoxy, *iso*-pentoxy, *sec*-pentoxy, *tert*-pentoxy, *neo*-pentoxy, *n*-hexoxy, *sec*- hexoxy, *iso*- hexoxy, *tert*-hexoxy, and *neo*-hexoxy

- **"alkoxycarbonyl"** refers to any -C(=O)-alkoxy group.

- **"alkylcarbonyl"** refers to any -C(=O)-alkyl group.

- **"heterocycliccarbonyl"** refers to any -C(=O)-heterocyclic group

- **"carbocycliccarbonyl"** refers to any -C(=O)-carbocyclic group

- **"alkylthio"** refers to any S-alkyl or S-carbocyclic group. In a preferred embodiment, the alkylthio group contains 1 to 4 carbon atoms.

- **"amino"** refers to any ammonia derivatives coming from the substitution of one or more hydrogen atoms by one or more organic radical(s). Examples of amino group include but are not limited to amine, amide, carbamate and hydroxylamine groups.

- **"carbamate"** refers to any -OC(=O)-R group, wherein R is alkyl, alkenyl, alkynyl, carbocyclic, heterocyclic, or haloalkyl.

- **"solvate"** is used herein to describe a molecular complex comprising the compound(s) of the invention and one or more pharmaceutically acceptable solvent molecules, for example, ethanol.

- **"subject",** alternatively referred to herein as "patient", refers to an animal, preferably a mammal, most preferably a human, who/which is awaiting or receiving medical care or is or will be the object of a medical procedure.

- **"human"** refers to subject of both genders and at any stage of development (i.e. neonate, infant, juvenile, adolescent, adult).

- **"treat", "treating"** and **"treatment,** as used herein, are meant to include reducing, alleviating or abrogating a condition or disease and/or its attendant symptoms.

- **"prevent", "preventing"** and **"prevention"** or "inhibiting the development of a disease", as used herein, refer to a method of delaying or precluding the onset of a condition or disease and/or its attendant symptoms, barring a patient from acquiring a condition or disease, or reducing a patient's risk of acquiring a condition or disease.

- **"therapeutically effective amount"** (or more simply an **"effective amount")** as used herein means the level or amount of agent (e. g. a compound of the invention) which is sufficient to achieve the desired therapeutic or prophylactic effect in the individual to which it is administered. Especially, it refers to the level or amount of agent that is aimed at, without causing significant negative or adverse side effects to the target,

  (1) delaying or preventing the onset of a disease, disorder, especially a neurodegenerative disease such as for example Alzheimer disease or a disease involving an activation of PDE4;
  (2) slowing down or stopping the progression, aggravation, or deterioration of one or more symptoms of a disease, disorder, or condition, especially a neurodegenerative disease, such as for example Alzheimer disease or a disease involving an activation of PDE4;
  (3) bringing about ameliorations of the symptoms of a disease, disorder, or condition, especially a neurodegenerative disease, such as for example Alzheimer disease or a disease involving an activation of PDE4;
  (4) reducing the severity or incidence of a disease, disorder, or condition, especially a neurodegenerative disease, such as for example Alzheimer disease or a disease involving an activation of PDE4; or
  (5) curing a disease, disorder, or condition , especially a neurodegenerative disease, such as for example Alzheimer disease or a disease involving an activation of PDE4.

- **"administering"** a compound should be understood to mean providing a compound of the invention alone or as part of a pharmaceutically acceptable composition to the patient in whom/which the condition, symptom, or disease is to be treated or prevented.

- **"pharmaceutically acceptable"** means that the ingredients of a pharmaceutical composition are compatible with each other and not deleterious to the patient.

- **"pharmaceutically acceptable compound"** refers to a compound that does not produce an adverse, allergic or other untoward reaction when administered to a patient. It includes any and all solvents, dispersion media, coatings, antibacterial and antifungal agents, isotonic and absorption delaying agents and the like. For human administration, preparations should meet sterility, pyrogenicity, general safety and purity standards as required by FDA Office of Biologics standards.

- **"pharmaceutically acceptable vehicle"** as used herein means a carrier or inert medium used as solvent or diluent in which the pharmaceutically active agent is formulated and/or administered. Non-limiting examples of pharmaceutical vehicles include creams, gels, lotions, solutions, and liposomes.

- **"pharmaceutical composition"** refers to a composition comprising an active principle in association with a pharmaceutically acceptable vehicle or excipient. A pharmaceutical composition is for therapeutic use, and relates to health. Especially, a pharmaceutical composition may be indicated for treating or preventing a disease.

- **"neurodegenerative disease"** refers to all and any disease where a progressive loss of structure or function of neurons, including death of neurons, occurs.

## DETAILED DESCRIPTION

This invention also relates to a compound of formula (I) below for use in the prevention and/or the treatment of a neurodegenerative disease, such as for example Alzheimer disease, Parkinson disease, multiple sclerosis or amyotrophic lateral sclerosis, especially Alzheimer disease or for the prevention and/or the treatment of a disease involving an activation of PDE4.

According to one embodiment, the compound of the invention, for use for preventing and/or treating a disease involving an activation of PDE4 is a modulator of PDE4, preferably an allosteric modulator of PDE4.

***Compounds for use according to the present invention.***

According to the invention, the compound for use in the prevention and/or treatment of a neurodegenerative disease or a disease involving an activation of phosphodiesterase-4, is a compound of general formula (I) below or a pharmaceutically acceptable salt or solvate thereof:

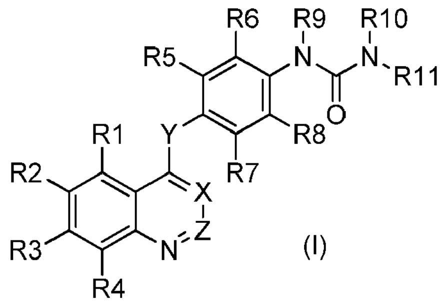

(I)

wherein X and Z each independently represent CH or N;
Y represents O or S;
R1, R2 and R3 may be the same or different and represent a hydrogen atom, a halogen atom, hydroxyl, nitro, cyano, amino, amido, carbamate, alkyl, alkenyl, alkynyl, alkoxy, heterocyclic, carbocyclic, alkylthio, carboxy, or alkoxycarbonyl;
R4 represent a hydrogen atom;
R5, R6, R7 and R8 may be the same or different and represent a hydrogen atom, halogen atom, hydroxyl, nitro, amino, alkyl, alkoxy, alkylthio, or trifluoromethyl;
R9 and R10 may be the same or different and represent a hydrogen atom, alkyl, alkylcarbonyl, heterocyclic, carbocyclic, $ONO_2$, or $OSO_2R$ wherein R is hydroxyl, alkyl, carbocyclic; and
R11 represents an alkyl, alkenyl, alkynyl, alkoxy, akoxycarbonyl, amino, amido, heterocyclic, carbocyclic, or optionally substituted azolyl.

In one embodiment, X represents CH. In another embodiment, X represents N.

In one embodiment, Z represents CH.

According to one embodiment, X and Z both represent CH. In another embodiment, X represents N and Z represents CH.

In one embodiment, Y represents O.

In one embodiment, R1, R2 and R3 are the same or different and are selected in the group comprising hydrogen atom, nitro, cyano, amino, alkyl, alkenyl, alkynyl, and alkoxy, preferably selected in the group comprising hydrogen atom, $C_{1-6}$ alkoxy and cyano.

In a preferred embodiment, R1 is H. In another preferred embodiment, R1 represents H and R2 and R3 are the same or different and independently represent methoxy, - $OCH_2CH_2OCH_3$, cyano, (1-aminocyclopropyl)methanolyl and (1-(dimethylamino)cyclopropyl)-methanolate. According to one embodiment, R2 and R3 both represent an optionally substituted $C_{1-6}$ alkoxy group, preferably $C_{1-4}$ alkoxy group, more preferably methoxy.

In an embodiment, R5, R6, R7, and R8 are the same or different and independently represent a hydrogen atom or a halogen atom. In a preferred embodiment, R5, R6, R7, and R8 are the same or different and independently represent a hydrogen, a chlorine or a fluorine atom.

In an embodiment, at least one of R5, R6, R7, and R8 is a halogen atom, preferably chlorine or fluorine atom. In one embodiment, R8 represents a halogen atom, preferably chlorine or fluorine atom, more preferably a chlorine atom.

In one embodiment, R5 represents H.

In one embodiment, R6 represents H.

In one embodiment, R7 represents H.

According to one embodiment, R5, R6 and R7 are hydrogen atoms.

According to one embodiment, R5, R6 and R7 are hydrogen atoms and R8 represents a halogen atom, preferably chlorine or fluorine atom, more preferably a chlorine atom.

In an embodiment, R9 and R10 are the same or different and represent a hydrogen atom, alkyl, alkylcarbonyl, $ONO_2$, $OSO_2R$, wherein R is hydroxyl, alkyl, or cycloalkyl

In one embodiment, R9 represents H.

In one embodiment, R10 represents H.

In a preferred embodiment, R9 and R10 are both hydrogen atoms.

In an embodiment, R11 is the group represented by the formula (i):

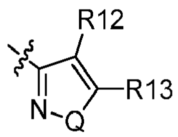

(i)

wherein Q represent O, S or NH; preferably Q represent O, and
R12
and R13 may be the same or different and represent hydrogen atom, halogen atom, alkyl, alkoxy, alkylthio, trifluoromethyl, nitro amino, alkoxycarbonyl, alkylcarbonyl, carbocyclic; preferably R12 and R13 may be the same or different and represent a hydrogen atom or a $C_{1-4}$ alkyl, more preferably a hydrogen atom or a methyl.

In an embodiment, R11 is the group represented by the formula (ii):

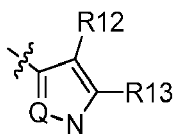

(ii)

wherein Q represents O, S or NH, and
R12 and R13 may be the same or different and represent a hydrogen atom, a halogen atom, alkyl, alkoxy, alkylthio, trifluoromethyl, nitro, amino, alkoxycarbonyl, alkylcarbonyl, or carbocyclic group; preferably R12 and R13 may be the same or different and represent hydrogen atom or a $C_{1-4}$ alkyl, more preferably a hydrogen atom or a methyl.

In an embodiment, R11 is the group represented by the formula (iii):

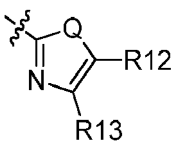

(iii)

wherein Q represents O, S or NH; preferably Q represent S, and
R12 and R13 may be the same or different and represent a hydrogen atom, a halogen atom, alkyl, alkoxy, alkylthio, trifluoromethyl, nitro, amino, alkoxycarbonyl, alkylcarbonyl, or carbocyclic; preferably R12 and R13 may be the same or different and represent a hydrogen atom; a chlorine atom; a bromine atom; methyl, t-butyl, acetyl or - $CH_2$-COOEt.

In an embodiment, R11 is the group represented by the formula (iv):

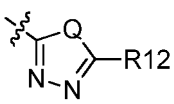

(iv)

wherein Q represents O, S or NH; preferably Q represent S, and
R12 represents a hydrogen atom, a halogen atom, alkyl, alkoxy, alkylthio, trifluoromethyl, nitro, amino or carbocyclic group ; preferably R12 represents methyl, ethyl, t-butyl, trifluoromethyl, ethylthio, or cyclopropyl.

In an embodiment, R13 is a hydrogen atom in groups (i) and (ii).

In an embodiment, R11 represents an azolyl, alkyl, amido, carbocyclic or heterocyclic groups, preferably an azolyl of formula (i), (ii), (iii) or (iv), $C_{3-10}$ carbocyclic, $C_{3-10}$ heterocyclic, amide or $C_{1-6}$ alkyl. In a preferred embodiment,

R11 is selected from *n*-propyl, 2-MeOPh, 4-MeOPh, 2-MePh, 1-naphthyl, 5-chloropyridin-2-yl, 5-bromopyridin-2-yl, 5-methylpyridin-2-yl, 2,5-dichlorophenyl, 2-chloro-5-bromophenyl, 2-fluoro-5-chlorophenyl, 2-methoxy-5-chlorophenyl, 2-methoxy-5-bromophenyl, 2-methyl-5-fluorophenyl, 2-(difluoromethoxy)-5-chlorophenyl, or 5-methylisoxazo-3-yle.

In a preferred embodiment, R11 represents *n*-propyl or isoxazole group, preferably 5-methylisoxazo-3-yle.

In an embodiment, compound for use of the invention is compound of formula (Ia) or a pharmaceutically acceptable salt or solvate thereof, wherein compound of formula (Ia) is represented hereunder:

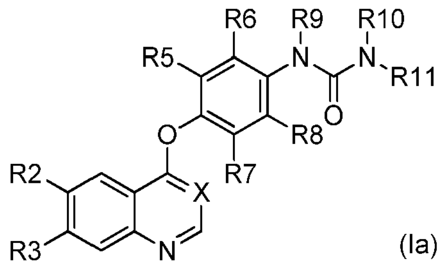

(Ia)

wherein R2, R3, R5, R6, R7, R8, R9, R10, R11, and X are as defined for compound of general formula I.

In an embodiment, the compound of formula (Ia) is such that:

X represents CH or N;
R2 and R3 may be the same or different and represent a hydrogen atom, a halogen atom, hydroxyl, nitro, cyano, amino, amido, carbamate, alkyl, alkenyl, alkynyl, alkoxy, heterocyclic, carbocyclic, alkylthio, carboxy, or alkoxycarbonyl;
R5, R6, R7 and R8 may be the same or different and represent a hydrogen atom, halogen atom, hydroxyl, nitro, amino, alkyl, alkoxy, alkylthio, or trifluoromethyl;
R9 and R10 may be the same or different and represent a hydrogen atom, alkyl, alkylcarbonyl, heterocyclic, carbocyclic, $ONO_2$, or $OSO_2R$ wherein R is hydroxyl, alkyl, carbocyclic; and
R11 represents an alkyl, alkenyl, alkynyl, alkoxy, akoxycarbonyl, amino, amido, heterocyclic, carbocyclic, or optionally substituted azolyl.

In one embodiment, X represents CH. In another embodiment, X represents N.

In an embodiment, R1 represents H and R2 and R3 are the same or different and independently represent methoxy, $-OCH_2CH_2OCH_3$, cyano, (1-aminocyclopropyl)methanolyl and (1-(dimethylamino)cyclopropyl)-methanolate. According to one embodiment, R2 and R3 both represent an optionally substituted $C_{1-6}$ alkoxy group, preferably $C_{1-4}$ alkoxy group, more preferably methoxy.

In one embodiment, R2 and R3 are the same or different and are selected in the group comprising hydrogen atom, nitro, cyano, amino, alkyl, alkenyl, alkynyl, and alkoxy, preferably selected in the group comprising hydrogen atom, $C_{1-6}$ alkoxy and cyano.

In preferred embodiment, R2 and R3 are the same or different and independently represent methoxy, $-OCH_2CH_2OCH_3$, cyano, (1-aminocyclopropyl)methanolyl and (1-(dimethylamino)cyclopropyl)-methanolate. According to one embodiment, R2 and R3 both represent an optionally substituted $C_{1-6}$ alkoxy group, preferably $C_{1-4}$ alkoxy group, more preferably methoxy.

In an embodiment, R5, R6, R7, and R8 are the same or different and independently represent a hydrogen atom or a halogen atom. In a preferred embodiment, R5, R6, R7, and R8 are the same or different and independently represent a hydrogen, a chlorine or a fluorine atom.

In an embodiment, at least one of R5, R6, R7, and R8 is a halogen atom, preferably chlorine or fluorine atom.

In one embodiment, R6 represents H.

In one embodiment, R7 represents H.

According to one embodiment, R5, R6 and R7 are hydrogen atoms.

In one embodiment, R8 represents a halogen atom, preferably chlorine or fluorine atom, more preferably a chlorine atom.

In one embodiment, R9 and R10 are the same or different and represent a hydrogen atom, alkyl, alkylcarbonyl, $ONO_2$, $OSO_2R$, wherein R is hydroxyl, alkyl, or cycloalkyl, preferably R9 and R10 are both hydrogen atoms.

In an embodiment, R11 is an optionally substituted azolyl, preferably of formula (i), (ii), (iii) or (iv) as presented above.

In an embodiment, R11 represents an azolyl, alkyl, amido, carbocyclic or heterocyclic groups, preferably an azolyl of formula (i), (ii), (iii) or (iv), $C_{3-10}$ carbocyclic, $C_{3-10}$ heterocyclic, amide or $C_{1-6}$ alkyl. In a particular embodiment,

R11 is selected from *n*-propyl, 2-MeOPh, 4-MeOPh, 2-MePh, 1-naphthyl, 5-chloropyridin-2-yl, 5-bromopyridin-2-yl, 5-methylpyridin-2-yl, 2,5-dichlorophenyl, 2-chloro-5-bromophenyl, 2-fluoro-5-chlorophenyl, 2-methoxy-5-chlorophenyl, 2-methoxy-5-bromophenyl, 2-methyl-5-fluorophenyl, 2-(difluoromethoxy)-5-chlorophenyl, or 5-methylisoxazo-3-yle.

In a preferred embodiment, R11 represents *n*-propyl or isoxazole group, preferably 5-methylisoxazo-3-yle.

In a preferred embodiment, compound (I) is N-{2-chloro-4-[(6,7-dimethoxy-4-quinolyl)oxy]-phenyl}-N'-(5-methyl-3-isoxazolyl)urea, also commonly named tizovanib (Av-951, KRN951), and represented by the following formula:

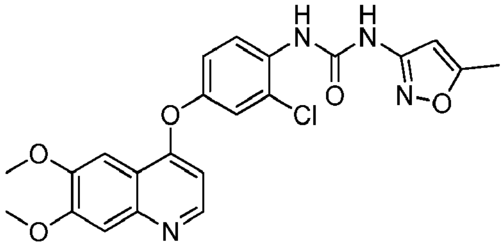

In a preferred embodiment, compound (I) is 1-(2-chloro-4-((6,7-dimethoxyquinazolin-4-yl)oxy)phenyl)-3-propylurea, and represented by the following formula:

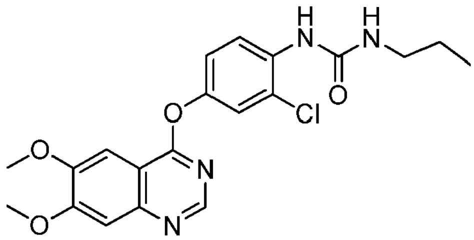

In an embodiment, the compound for use of the present invention may be used under the form of pharmaceutically acceptable salts thereof. Preferred examples of such salts include: alkali metal or alkaline earth metal salts such as sodium salts, potassium salts or calcium salts; hydrohalogenic acid salts such as hydrofluoride salts, hydrochloride salts, hydrobromide salts, or hydroiodide salts; inorganic acid salts such as nitric acid salts, perchloric acid salts, sulfuric acid salts, or phosphoric acid salts; lower alkylsulfonic acid salts such as methanesulfonic acid salts, trifluoromethanesulfonic acid salts, or ethanesulfonic acid salts; arylsulfonic acid salts such as benzenesulfonic acid salts or p-toluenesulfonic acid salts; organic acid salts such as fumaric acid salts, succinic acid salts, citric acid salts, tartaric acid salts, oxalic acid salts, maleic acid salts, acetic acid salts, malic acid salts, lactic acid salts, or ascorbic acid salts; and amino acid salts such as glycine salts, phenylalanine salts, glutamic acid salts, or aspartic acid salts.

***Use for the prevention and/or treatment of a disease involving an activation of PDE4***

Compounds of the present invention are for use in the prevention and/or treatment of diseases involving an activation of PDE4.

According to one embodiment, said diseases involving an activation of PDE4 may be neurological diseases, neurodegenerative diseases, inflammatory diseases, respiratory diseases, allergies, cardiovascular diseases, thrombosis, diabetes, cancer, rheumatoid arthritis, atopic dermatitis, psoriasis, asthma, inflammatory bowel disease.

According to an embodiment, the disease involving an activation of PDE4 is a neurodegenerative disease. According to an embodiment, the neurodegenerative disease is Parkinson disease, Huntington disease, Alzheimer disease, multiple sclerosis, Amyotrophic Lateral Sclerosis, Spinal Muscular Atrophy or any disease wherein a progressive loss of structure or function of neurons, including death of neurons, occurs. In a preferred embodiment, the neurodegenerative disease is Alzheimer's disease.

According to an embodiment, the disease involving an activation of PDE4 is cancer. According to an embodiment, the cancer is a brain cancer, lung cancer, leukemia for example chronic lymphocytic leukaemia, osteosarcoma, and melanoma.

.In an embodiment, a therapeutically effective amount of compound for use of the invention may be administered prior to the onset of a disease, disorder, or condition involving an activation of PDE4. In an embodiment, a therapeutically effective amount of compound for use of the invention may be administered prior to the onset of a disease, disorder, or condition related to neurodegenerative disease, especially Alzheimer disease, for a prophylactic or preventive action.

In an embodiment, the therapeutically effective amount of compound of the invention may be administered

after initiation of a disease, disorder, or condition involving an activation of PDE4, preferably a disease, disorder, or condition related to neurodegenerative disease, especially Alzheimer disease, for a therapeutic action.

In an embodiment, the therapeutically effective amount of compound of the invention may be administered prior to and after initiation of a disease, disorder, or condition involving an activation of PDE4, preferably a disease, disorder, or condition related to neurodegenerative disease, especially Alzheimer disease, for a prophylactic, preventive and/or therapeutic action.

In an embodiment, the compounds for use of the present invention have an *in vitro* activity against PDE4. In an embodiment, tivozanib has an *in vitro* activity against PDE4.

In an embodiment, a therapeutically effective amount of compound of the invention is an amount that is effective in reducing at least one symptom of at least one neurodegenerative disease, especially Alzheimer disease.

In an embodiment, the therapeutically effective amount of compound may be appropriately determined in consideration of, for example, the age, weight, sex, difference in diseases, and severity of the condition of individual patients. It will be understood, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention further relates to a pharmaceutical composition comprising the compound for use according to the present invention, or salt or solvate thereof, in association with a pharmaceutically acceptable vehicle. The pharmaceutical composition of the invention is for use in the prevention and/or treatment of a disease selected from the group consisting of a neurodegenerative disease and a disease involving an activation of PDE4.

In an embodiment, the compound for use of the invention or the pharmaceutical composition of the invention is administered to human or non-human animals.

In an embodiment, the compound for use of the invention or the pharmaceutical composition of the invention is administered orally. In this embodiment, oral preparations include tablets, capsules, powders, granules, and syrups.

In another embodiment, the compound for use of the invention or the pharmaceutical composition of the invention is administered parenterally, for example by intravenous injection, intramuscular injection, subcutaneous injection, intradermic injection, intraperitoneal injection, intracerebroventrocular (ICV) infusion, intracisternal injection or infusion.

In another embodiment, the compound for use of the invention or the pharmaceutical compositions of the invention is administered by inhalation spray, nasal, vaginal, rectal, sublingual, percutaneous or topical routes of administration.

In one embodiment, the compounds for use of the invention may be formulated, alone or together, in suitable dosage unit formulations containing conventional non-toxic pharmaceutically acceptable carriers, adjuvants and vehicles appropriate for each route of administration.

In an embodiment, these various preparations may be prepared by conventional methods, for example, with commonly used pharmaceutically acceptable carriers, such as excipients, disintegrants, binders, lubricants, colorants, and diluents.

In an embodiment, excipients include, for example lactose, glucose, corn starch, sorbit, and crystalline cellulose.

In an embodiment, disintegrants include, for example starch, sodium alginate, gelatin powder, calcium carbonate, calcium citrate, and dextrin.

In an embodiment, binders include, for example dimethylcellulose, polyvinyl alcohol, polyvinyl ether, methylcellulose, ethylcellulose, gum arabic, gelatin, hydroxypropylcellulose, and polyvinyl pyrrolidone.

In an embodiment, lubricants include, for example talc, magnesium stearate, polyethylene glycol, and hydrogenated vegetable oils.

In an embodiment, the pharmaceutical composition of the invention may further contain preparing buffers, pH adjusters, stabilizers, tonicity agents, and/or preservatives.

In an embodiment, the content of the compound according to the invention in the pharmaceutical composition according to the invention may vary depending on the dosage form.

In an embodiment, the compound according to the present invention may be administered without any other active ingredients which are usually applied in the prevention and/or treatment of the above mentioned pathological conditions.

In an embodiment, the pharmaceutical composition of the present invention may further comprise other therapeutically active compounds which are usually applied in the prevention and/or treatment of the above mentioned pathological conditions.

In an embodiment, the pharmaceutical compositions for the administration of the compounds for use of this invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by

uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

In one embodiment, the pharmaceutical compositions of the invention is in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions

In one embodiment, the pharmaceutical compositions are in the form of a sterile injectable aqueous or oleaginous suspension. This suspension may be formulated according to the known art using suitable dispersing or wetting agents and suspending agents.

In one embodiment, the compounds of the present invention are administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug.

For topical use, creams, ointments, jellies, solutions or suspensions, tapes etc., containing the compounds of the present invention may be employed.

The invention further relates to a method of prevention and/or treatment of a subject suffering from a neurodegenerative disease and/or a disease involving an activation of phosphodiesterase-4, said method comprising administering to that patient a therapeutic effective amount of a compound as described in this invention or a pharmaceutically acceptable salt thereof. Preferably the compound is of formula (I) or (Ia). More preferably, the compound is tivozanib.

In one embodiment, compounds of the invention may be used as psycho-stimulants, analgesics, antipyretics, psycholeptics or anxiolitics in the prevention and/or treatment of diseases involving an activation of PDE4.

## BRIEF DESCRIPTION OF THE DRAWINGS

**Figure 1** is a picture showing the building of a spherical harmonic based shape of a cholesterol like compound.

**Figure 2** is a picture showing a spherical harmonic based shape of PDE4 modulator D159687.

**Figure 3** is a picture showing a pharmacophoric representation of the PDE4 modulator D159153.

**Figure 4** is a graph showing the biochemical activity curve for tivozanib against PDE4.

**Figure 5** is a SDS-PAGE analysis of Aβ oligomers used in the study of in-vitro activity of Tivozanib.

**Figure 6** is a graph representing the effects of tivozanib on Aβ oligomer-induced neuronal death.

**Figure 7** is a graph representing the effects of tivozanib on cAMP level.

**Figure 8** is a graph representing tivozanib preventing Aβ oligomer-induced impairment of short term memory.

## EXAMPLES

The present invention is further illustrated by the following examples.

### <u>Example 1</u> ***In vitro*** **activity of tivozanib against PDE4 isolated enzyme PDE Assay**

PDE activities are measured by a two-step radioenzymatic assay as previously described at a substrate concentration of 1 μM cAMP or cGMP in the presence of 15,000 cpm [3H]-cAMP or [3H]-cGMP as a tracer, respectively. During the first incubation (30°C for 30 min) performed in a final volume of 250 μL, cyclic nucleotides are hydrolyzed into their respective nucleotide monophosphates. This incubation is stopped by the addition of an excess of cAMP and cGMP in the presence of EDTA and a nonselective PDE inhibitor (theophylline) that inactivates PDE activity. The second incubation performed in the presence of an excess of 5'-nucleotidase (snake venom) results in the formation of adenosine

or guanosine. This second incubation is stopped by the addition of an excess of adenosine and guanosine in the presence of EDTA. The enzymatic reaction products are separated by anion-exchange column chromatography on QAE-Sephadex A-25 formate. The amount of resulting [3H]-nucleoside is determined by liquid scintillation.

**Purification of Radiolabeled cAMP or cGMP**

[3H]-cAMP and [3H]-cGMP are purified by TLC on Silicagel 60 F254 TLC plates.

1. Along a horizontal line drawn 2 cm from the bottom of a TLC plate, apply 2 μL of 10 mM unlabeled cyclic nucleotides as migration markers.
2. Separately spot 80 μL of [3H]-cAMP and [3H]-cGMP by successive additions of 10 μL under drying air.
3. Place the TLC plate in a vertical developing tank containing TLC chromatography solvent and develop for approx 4 h, until the solvent front is 4 cm beneath the top of the plate. Then dry the plate.
4. Under an ultraviolet light (254 nm), locate the positions of the cyclic nucleotide standards, as well as those of the labeled cyclic nucleotides, and surround with pencil. Using a razor blade, scrape off the corresponding region for each labelled cyclic nucleotide onto aluminum paper, and transfer the silicagel powder into a 1.5-mL Eppendorf tube.
5. To extract the cyclic nucleotide, add 1 mL of 50% ethanol to the tube, thoroughly mix, and centrifuge to settle down the silicagel; repeat this extraction a second time with 0.4 mL. Then combine the supernatants.
6. Count a sample (10 μL) of the purified labeled cyclic nucleotide. Adjust the supernatant with 50% ethanol to give 15,000 cpm/μL and store at -20°C.

**QAE-Sephadex A-25 Formate Anion-Exchange Resin (12)**

The use of anion-exchange resin to recover [3H]-adenosine or [3H]-guanosine is critical for the accuracy of the assay. This method allows recovering and regenerating the resin, which can then be used over a long period of time. Because QAE-Sephadex A-25 is expensive, the ability to recycle is quite valuable.

**Preparation and equilibration of resin**

1. Allow 250 g of QAE-Sephadex A-25 (in the chloride form) to swell in 5 L of Millipore water overnight at 4°C.
2. Convert the resin from the OH- form to the formate form using steps 3-8 in Subheading REGENERATION OF RESIN
3. Store the resin at +4°C as a 50% slurry.

**Regeneration of resin**

Used resin collected from the assay columns is stored at +4°C and regenerated in a large glass column (35 × 8 cm) with a sintered-glass bottom by successive treatments:

1. Treat with 0.1 N HCl until the pH of the effluent is 1.0 to 2.0.
2. Treat with Millipore water until the pH of the effluent is 5.0 to 6.0.
3. Treat with 0.2 N NaOH until the effluent is Cl- free, as checked with AgN03 under acidic conditions.
4. Treat with Millipore water until the pH of the effluent is 6.0 to 7.0.
5. Treat with 0.2 N formic acid until the pH of the effluent is about 2.5.
6. Treat with 2 M ammonium formate until the pH of the effluent is 6.4 to 6.7.
7. Treat with Millipore water until the pH of the effluent is about 4.0.
8. Suspend the resin in 100 mM ammonium formate and store at 4°C.

**Two-Step PDE Assay**

**ENZYMATIC REACTIONS**

Adding reactive constituents sequentially by 25- or 50-μL fractions constitutes the 250-μL final assay medium, allowing maximum flexibility and convenience for several assay purposes. During this procedure test tubes are kept in an ice-water bath.

1. Add 25 μL of Millipore water, solvent, or drug (made up soluble with solvent such as dimethyl sulfoxide [DMSO] or ethanol) at 10X concentration to be tested.
2. Add 25 μL of Millipore water or additional drug.
3. Add 25 μL of Tris-Mg-Ca.
4. Add 25 μL of Mg-EGTA or Mg-CaM.
5. Add 50 μL of Tris-BSA.
6. 50 μL of tissue homogenate (PDE extract) diluted with Tris-BSA to give about 15% of the substrate hydrolyzed in control conditions
7. Vortex the test tubes.
8. Initiate the enzymatic reaction by adding 50 μL of substrate solution (25 μL of 10 μM cAMP or cGMP + 24 μL of Millipore water + 1 μL of purified [3H]-cAMP or [3H]-cGMP, 15,000 cpm), gently vortex, and incubate at 30°C.
9. Stop the reaction by adding 25 μL of PDE-stop, and after vortexing the test tubes, return them to the ice-water bath.
10. Initiate the nucleotidase reaction by adding 20 μL of SV; vortex the tubes and incubate for 20 min at 30°C.
11. Stop the reaction by adding 300 μL of SV-stop.

**QAE-SEPHADEX FORMATE ANION-EXCHANGE COLUMN CHROMATOGRAPHY**

This procedure allows separation of the dephosphorylated products (adenosine or guanosine) from the nonhydrolyzed cyclic nucleotide.

1. For each test tube, fill a chromatography column with 2 mL of 50% resin slurry (see Subheading QAE-Sephadex) in order to get 1 mL of settled resin.
2. Equilibrate the columns by adding 3 mL of formate solution.
3. By using a homemade rack, place each column above a scintillation vial.
4. Pour the test tubes onto their corresponding columns.
5. Rinse the test tubes with 1.5 mL of formate solution and pour again onto the columns.
6. Add 7 mL of scintillation fluid to each vial, cap the vials, vigorously vortex, and then place in a β liquid scintillation counter.
7. Recover the resin from the columns and store at +4°C until regeneration; wash the columns exhaustively with hot tap water.

**Expression of Data**

To determine PDE activity, four test tubes without PDE extract (50 μL of Tris-BSA) are run in each assay procedure until step 11 in Subheading ENZYMATIC REACTIONS. Then the two first tubes, used to determine the total radioactivity (Rt), are directly poured into scintillation vials and 1.5 mL of formate is added; the two other tubes, used to determine blank assay (Ro), are treated similarly to the other test tubes.
The percentage of cyclic nucleotide phosphate (CNP) hydrolyzed is calculated as follows:

% CNP hydrolyzed = Rx-Ro / RT-Ro, wherein Rx is the cpm of the sample, Ro is the cpm of the blank assay, and RT is the cpm of the total radioactivity.

The PDE activity of the sample is calculated as follows:

$$\text{PDE activity (pmol/[min}\bullet\text{mg]) } =(\%\ \text{CNP})(0.25\times 10\text{–}3)(1\times 10^{-6}\text{M}) \ /\ t \times(0.050\text{C}),$$

wherein t is the incubation time of the first enzymatic reaction (min), and C is the protein concentration (mg/mL).
The inhibition concentration value (IC50) of tivozanib is of 4.8μM in this assay. The results are presented in figure 4.

**<u>Examule 2</u>: *In vitro* activity of tivozanib against PDE4 on cell culture**

*Material and Methods*

<u>Cell culture</u>: Cortical neurons from embryonic day 16-17 Wistar rat fetuses were prepared as follows. Dissociated cortical cells were plated at 4.5-5.0 $10^4$ cells/$cm^2$ in plastic dishes precoated with 1.5 mg/mL polyornithine (Sigma). Cells

were cultured in a chemically defined Dulbecco's modified eagle's/F12 medium free of serum (Gibco) and supplemented with hormones, proteins and salts. Cultures were kept at 35°C in a humidified 5% $CO_2$ atmosphere, and at 6-7 DIV, cortical population was determined to be at least 97% neuronal by immunostaining.

Preparation of low-n β-amyloid (Aβ) oligomers: The peptide used is $A\beta_{1-42}$ from Bachem (ref H1368, batch number: 1025459). The oligomeric preparation contains a mixture of stable dimers, trimers and tetramers of $A\beta_{1-42}$ peptide, as well as monomeric forms of the peptide.

The same preparation of oligomers was used for all experimental set-ups and has been previously characterized in terms of oligomer composition (see figure 5), neurotoxicity *in vitro* as well as induction of cognitive impairment in mice.

Preparation of tivozanib solution: Tivozanib was dissolved in DMSO. A stock solution (10 mM DMSO) was prepared, and aliquots were stored at -20 °C before use.

Treatment and measurement of cell viability: All experiments were done in 48 well plates. At DIV 6, primary cortical neurons were incubated with vehicle or increasing concentrations of tivozanib (up to 10 μM) in the absence or presence of 1 μM low-n $A\beta_{1-42}$ oligomers. Following 24h of incubation, neuronal viability was monitored using the MTT assay (Garcia et al., The Journal of Neuroscience, June 2,2010, 30(22), 7516-7527; Desbene et al., Neurobiology of Aging, 33 (2012) 1123.e17-1123.e-29). Data were obtained from one experiment with 4 determinations each and expressed as means + SD.

Measurement of cAMP level: HEK293 cells were cultured in DMEM medium containing 10 % FCS. Cells were plated in 12-well plates at a density of 400.000 cells per well and cultured for 24h at 37°C in a humidified 6% $CO_2$ atmosphere. Cells were then incubated for 1 h with increasing concentrations of tivozanib and then treated with vehicle or 25 μM forskolin for 15 minutes. Following washing steps and cell lysis, cellular level of cAMP level was monitored using an ELISA assay (Cyclic AMP XP assay kit - Cell signaling). Data are from 2 experiments performed in duplicate. Data are represented as fold increase of cAMP as compared to experiment using forskolin only, designed as 1.0 (figure 7).

*Results*

Effects of tivozanib on low-n Aβ oligomer induced neuronal death: Rat cortical neurons were exposed for 24 h with vehicle or 1 μM $A\beta_{1-42}$ oligomers in the absence or presence of increasing concentration of tivozanib added simultaneously with the oligomer. The Aβ oligomer-induced neurotoxicity was evaluated using the MTT assay and results are presented in figure 6. In figure 6, graph A represents absorbance means ± SD (n = 4 per condition, 1 independent experiment) in MTT assay. Graph B represents cell viability as a percentage of control experiment designed as 100%. The incubation of cortical neurons with 1 μM $A\beta_{1-42}$ oligomers resulted in a ~40% decrease of cell viability (figure 6B, CTRL). Interestingly, the presence of tivozanib protected cells towards Aβ oligomer-induced cell death.

Effects of tivozanib on cAMP level in HEK293 cells: In order to evaluate the potential effect of tivozanib on PDE4 activity in living cells, the HEK293 cell line was used. These cells are widely used for identification and validation of PDE4 modulators in mild as well as high throughput screening assays. Indeed, the control of cAMP level and hydrolysis in HEK293 cells are predominantly supported by PDE4.

In control conditions (e.g. in the absence of stimulation of cAMP production), the levels of cAMP were low. The presence of tivozanib did not significantly impact cAMP level. By contrast, when cells were incubated with forskolin, an activator of adenylate cyclase, the level of cAMP was significantly increased. The incubation of cells with tivozanib at increasing concentrations (up to 50 μM) in the absence of forskolin did not result in an increase of cAMP level.

Interestingly, HEK293 cells incubated with increasing concentrations of tivozanib in the presence of forskolin exhibited a dose-response accumulation of cAMP (figure 7). These data show that tivozanib is able to inhibit cAMP hydrolysis following activation of adenylate cyclase by forskolin. This experiment shows that tivozanib interferes with cAMP metabolism through the modulation of PDE activity, most specifically PDE4-associated hydrolysis of cAMP.

*Conclusion*

These *in vitro* data shows that tivozanib prevents from soluble low-n oligomer-induced neuronal cell death. Moreover, tivozanib is able to improve cellular cAMP level in living cells, showing that cellular signaling pathway(s) induced by tivozanib modulates the activity of PDE4.

**Example 3: *In vivo* activity of tivozanib against PDE4**

*Material and Methods*

Animals: All animal experiments were carried out according to the National Institute of Health (NIH) guidelines for the care and use of laboratory animals, and approved by the French Ministry for Research and Technology. Animals were housed at a standard temperature (22±1°C) and in a light-controlled environment (lights on from 7 am to 8 pm)

with *ad libitum* access to food and water. Upon arrival, Male C57BL/6 mice (C57BL/6J Rj, ref SC-CJ-12w-M, Janvier, France) were group housed. From 1 week before the experiment started and until the end of the experiment, mice were individually housed. Animals were monitored twice-a-day by laboratory personnel (8 am and 4 pm). In case general health status of an animal is significantly worsens, the mouse was sacrificed. Definitions of acceptable endpoints are as follow: no spontaneous movements and inability to drink or eat in 24-h observation period, massive bleeding, spontaneous inflammation (general observations of mouse skin and eyes), or missing anatomy.

Surgery and intracerebroventricular (icv) infusion of low-n Aβ oligomers: Immediate spatial working memory performance was assessed by recording spontaneous alternation behavior in a Y-maze system, as previously described (Youssef et al., Neurobiology of Aging, 29 (2008) 1319-1333; Garcia et al., The Journal of Neuroscience, June 2,2010, 30(22) 7516-7527). Mice receiving a single stereotaxic icv injection of Aβ oligomers (50 pmol in 1 μL) developed memory deficits associated with an early decrease of hippocampal synaptic protein levels. Under anesthetization, soluble Aβ oligomers (50 pmol in 1 ml) or vehicle (saline, 1 ml) were injected into the right ventricle. The stereotaxic coordinates from the bregma were as follow (in mm): AP-0.22, L-1.0 and D-2.5. Injections were made using a 10 ml Hamilton microsyringe fitted with a 26-gauge needle.

Investigation of the spatial working memory - Y maze test: Immediate spatial working memory performance was assessed by recording spontaneous alternation behavior in a Y-maze system, as previously described (Youssef et al., 2008; Garcia et al., 2010, see references above). Mice were brought to the experimental room for at least 30 min acclimation to the experimental room conditions prior to testing. The maze was made of opaque Plexiglas and each arm were 40 cm long, 16 cm high, 9 cm wide and positioned at equal angles. The apparatus was placed in a test room homogeneously brightened in order to obtain 12-15 lux in arms as well as in the central zone. Mice were placed at the end of one arm and allowed exploring freely the maze during a 5 min session. The series of arm entries were recorded manually and the arm entry was considered to be completed when the hind paws of the mouse are completely placed in the arm. Alternation is defined as successive entries into the 3 arms on overlapping triplet sets. The percentage of alternation was calculated as the ratio of actual (total alternations) to possible alternations (defined as the number of arm entries minus 2), multiplied by 100.

Formulation of tivozanib and animal treatment: In order to administrate tivozanib through *per os* gavage, a 3 mg/mL tivozanib solution was prepared in 0.5% (w/v) methylcellulose. The solution was stirred (magnetic stirring) overnight protected from light. Animals were feed with tivozanib at a dose of 30 mg/kg/day from day -2 to day +4. At day 0, soluble Aβ low-n oligomers were icv administrated (induction of the disease) and spatial working memory performances measured at day +4 using the Y-maze test.

The different experimental groups (10 mice per group) are defined as follows:

- Group A: vehicle *per os* and vehicle icv
- Group B: vehicle *per os* and Aβ icv
- Group C: tivozanib *per os* and vehicle icv
- Group D: tivozanib *per os* and Aβ icv.

Statistical analysis: All data are presented as mean ± standard error of mean (SEM), and differences are considered to be statistically significant at the P<0.05 level. Statistical analysis is performed using Statview statistical software. Differences among means are analyzed by using 1-way-ANOVA followed by an appropriate post hoc test. Non-parametric data is analyzed with Kruskal-Wallis ANOVA.

*Results*

Effects of tivozanib on low-n Aβ oligomer-induced spatial working memory impairment: Spontaneous alternation behavior, which is considered to be a measure of immediate (short-term) spatial working memory performance, was investigated using the Y-maze test performed 4 days after icv injection of saline or Aβ oligomers. Spontaneous alternation (A) and the number of arm entries (B) were measured during a 5 min session (figure 8). Data are presented as mean ± S.E.M. p < 0.05 vs. Aβ-treated mice.

Mice receiving a vehicle gavage and icv injected with 50 pmol $A\beta_{1-42}$ oligomers displayed significantly impaired spatial working memory (p<0.05 as compared to mice receiving a vehicle gavage and icv injected with vehicle) (figure 8A).

Interestingly, tivozanib significantly improved spatial working memory as mice receiving tivozanib by gavage and icv injected with Aβ oligomers (group D) are not statistically different from control group A (vehicle gavage and vehicle icv injection) (figure 8A). Moreover, animals from group C (receiving tivozanib by gavage and icv injected with vehicle) displayed a normal behavior and their performances are not statistically different from groups A and D.

These data show that tivozanib protects mice from Aβ oligomer-induced impairment of short-term memory in an absence of pro-cognitive effects of the compound.

It is worthy to note that the number of entries of the different experimental groups are not statistically different,

showing that all treatments do not effect general behavior of the animals (e.g. motivation, locomotion in the apparatus) (figure 8B). Thus, the changes in alternation behavior are not due to generalized exploratory, locomotor or motivational effects.

*Conclusion*

These data show that tivozanib prevents from impairment of short-term memory in a preclinical mouse model for early AD.

## Claims

1. A compound for use in the prevention and/or treatment of a neurodegenerative disease or a disease involving an activation of phosphodiesterase-4, wherein said compound is of general formula (I) or a pharmaceutically acceptable salt or solvate thereof:

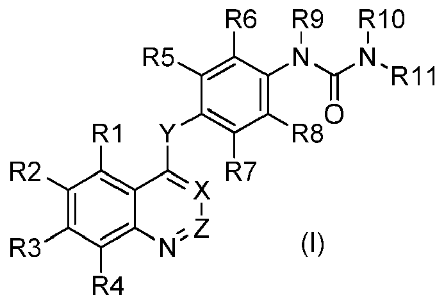

(I)

wherein X and Z each independently represent CH or N;
Y represents O or S;
R1, R2 and R3 may be the same or different and represent a hydrogen atom, a halogen atom, hydroxyl, nitro, cyano, amino, amido, carbamate, alkyl, alkenyl, alkynyl, alkoxy, heterocyclic, carbocyclic, alkylthio, carboxy, or alkoxycarbonyl;
R4 represent a hydrogen atom;
R5, R6, R7 and R8 may be the same or different and represent a hydrogen atom, halogen atom, hydroxyl, nitro, amino, alkyl, alkoxy, alkylthio, or trifluoromethyl;
R9 and R10 may be the same or different and represent a hydrogen atom, alkyl, alkylcarbonyl, heterocyclic, carbocyclic, $ONO_2$, or $OSO_2R$ wherein R is hydroxyl, alkyl, carbocyclic; and
R11 represents an alkyl, alkenyl, alkynyl, alkoxy, akoxycarbonyl, amino, amido, heterocyclic, carbocyclic, or optionally substituted azolyl.

2. A compound for use according to claim **1** or claim **2,** wherein the compound is of general formula (Ia) or a pharmaceutically acceptable salt or solvate thereof:

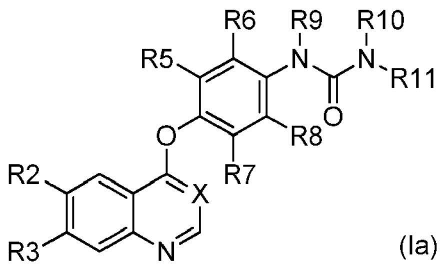

(Ia)

wherein X represents CH or N;
R2 and R3 may be the same or different and represent a hydrogen atom, a halogen atom, hydroxyl, nitro, cyano, amino, amido, carbamate, alkyl, alkenyl, alkynyl, alkoxy, heterocyclic, carbocyclic, alkylthio, carboxy, or alkoxycarbonyl;
R5, R6, R7 and R8 may be the same or different and represent a hydrogen atom, halogen atom, hydroxyl, nitro, amino, alkyl, alkoxy, alkylthio, or trifluoromethyl;
R9 and R10 may be the same or different and represent a hydrogen atom, alkyl, alkylcarbonyl, heterocyclic, carbocyclic, $ONO_2$, or $OSO_2R$ wherein R is hydroxyl, alkyl, carbocyclic; and
R11 represents an alkyl, alkenyl, alkynyl, alkoxy, akoxycarbonyl, amino, amido, heterocyclic, carbocyclic, or optionally substituted azolyl.

**3.** A compound for use according to Claim **1** or Claim **2,** wherein the azolyl group is of formula (i):

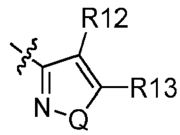

(i)

wherein Q represent O, S or NH; preferably Q represent O, and
R12 and R13 may be the same or different and represent hydrogen atom, halogen atom, alkyl, alkoxy, alkylthio, trifluoromethyl, nitro amino, alkoxycarbonyl, alkylcarbonyl, carbocyclic; preferably R12 and R13 may be the same or different and represent a hydrogen atom or a $C_{1-4}$ alkyl, more preferably a hydrogen atom or a methyl.

**4.** A compound for use according to Claim **1** or Claim **2,** wherein the azolyl group is of formula (ii):

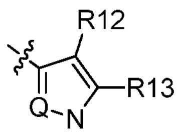

(ii)

wherein Q represents O, S or NH, and
R12 and R13 may be the same or different and represent a hydrogen atom, a halogen atom, alkyl, alkoxy, alkylthio, trifluoromethyl, nitro, amino, alkoxycarbonyl, alkylcarbonyl, or carbocyclic group; preferably R12 and R13 may be the same or different and represent hydrogen atom or a $C_{1-4}$ alkyl, more preferably a hydrogen atom or a methyl.

**5.** A compound for use according to Claim **1** or Claim **2,** wherein the azolyl group is of formula (iii):

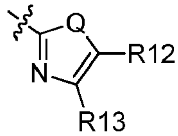

(iii)

wherein Q represents O, S or NH; preferably Q represent S, and
R12 and R13 may be the same or different and represent a hydrogen atom, a halogen atom, alkyl, alkoxy, alkylthio, trifluoromethyl, nitro, amino, alkoxycarbonyl, alkylcarbonyl, or carbocyclic; preferably R12 and R13 may be the same or different and represent a hydrogen atom; a chlorine atom; a bromine atom; methyl, t-butyl, acetyl or - $CH_2$-COOEt.

**6.** A compound for use according to Claim **1** or Claim **2,** wherein the azolyl group is of formula (iv):

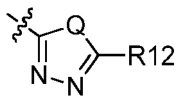

(iv)

wherein Q represents O, S or NH; preferably Q represent S, and
R12 represents a hydrogen atom, a halogen atom, alkyl, alkoxy, alkylthio, trifluoromethyl, nitro, amino or carbocyclic group ; preferably R12 represents methyl, ethyl, t-butyl, trifluoromethyl, ethylthio, or cyclopropyl.

7. A compound for use according anyone of claims **1** to **6,** wherein R1 represents a hydrogen atom and R2 and R3 are the same or different and independently represent methoxy, $-OCH_2CH_2OCH_3$, cyano, (1-aminocyclopropyl) methanolyl or (1-(dimethylamino)cyclopropyl)-methanolate, preferably both represent an optionally substituted $C_{1-6}$ alkoxy group, more preferably $C_{1-4}$ alkoxy group, and even more preferably a methoxy.

8. A compound for use according to anyone of claims **1** to **7,** wherein R5, R6, R7, and R8 are the same or different and independently represent a hydrogen atom or a halogen atom, preferably a chlorine or a fluorine atom, more preferably at least one of R5, R6, R7, and R8 is a halogen atom, preferably chlorine or fluorine atom, and even more preferably R5, R6 and R7 represent a hydrogen atom and R8 represents a halogen atom, preferably chlorine or fluorine atom, more preferably a chlorine atom.

9. A compound for use according to anyone of claims **1** to **8,** wherein R9 and R10 are the same or different and represent a hydrogen atom, alkyl, alkylcarbonyl, $ONO_2$, $OSO_2R$ wherein R is hydroxyl, alkyl, or cycloalkyl, preferably R9 and R10 are both hydrogen atoms.

10. A compound for use according to anyone of claims **1** to **9,** wherein R11 represents an azolyl, alkyl, amido, carbocyclic or heterocyclic groups, preferably an azolyl of formula (i), (ii), (iii) or (iv), $C_{3-10}$ carbocyclic, $C_{3-10}$ heterocyclic, amide or $C_{1-6}$ alkyl, more preferably, R11 is selected from *n*-propyl, 2-MeOPh, 4-MeOPh, 2-MePh, 1-naphthyl, 5-chloropyridin-2-yl, 5-bromopyridin-2-yl, 5-methylpyridin-2-yl, 2,5-dichlorophenyl, 2-chloro-5-bromophenyl, 2-fluoro-5-chlorophenyl, 2-methoxy-5-chlorophenyl, 2-methoxy-5-bromophenyl, 2-methyl-5-fluorophenyl, 2-(difluoromethoxy)-5-chlorophenyl, or 5-methylisoxazo-3-yle, and even more preferably R11 represents *n*-propyl or isoxazole group, preferably 5-methylisoxazo-3-yle.

11. A compound for use according to anyone of claims **1** to **10,** wherein the compound is N-{2-chloro-4-[(6,7-dimethoxy-4-quinolyl)oxy]-phenyl}-N'-(5-methyl-3-isoxazolyl)urea or 1-(2-chloro-4-((6,7-dimethoxyquinazolin-4-yl)oxy)phenyl)-3-propylurea.

12. A pharmaceutical composition comprising the compound for use according to anyone of claim **1** to **11** in association with a pharmaceutically acceptable vehicle.

13. A pharmaceutical composition according to claim **12,** for use in the prevention and/or treatment of a disease selected from the group consisting of a neurodegerative disease and a disease involving an activation of phosphodiesterase-4.

14. A compound for use according to anyone of claim **1** to **11** or a pharmaceutical composition according to claim **12** or claim **13,** wherein the disease is a neurodegenerative disease, including Alzheimer disease, Parkinson disease, Huntington disease, multiple sclerosis, amyotrophic lateral sclerosis, spinal muscular atrophy or any disease wherein a progressive loss of structure or function of neurons, including death of neurons, occurs.

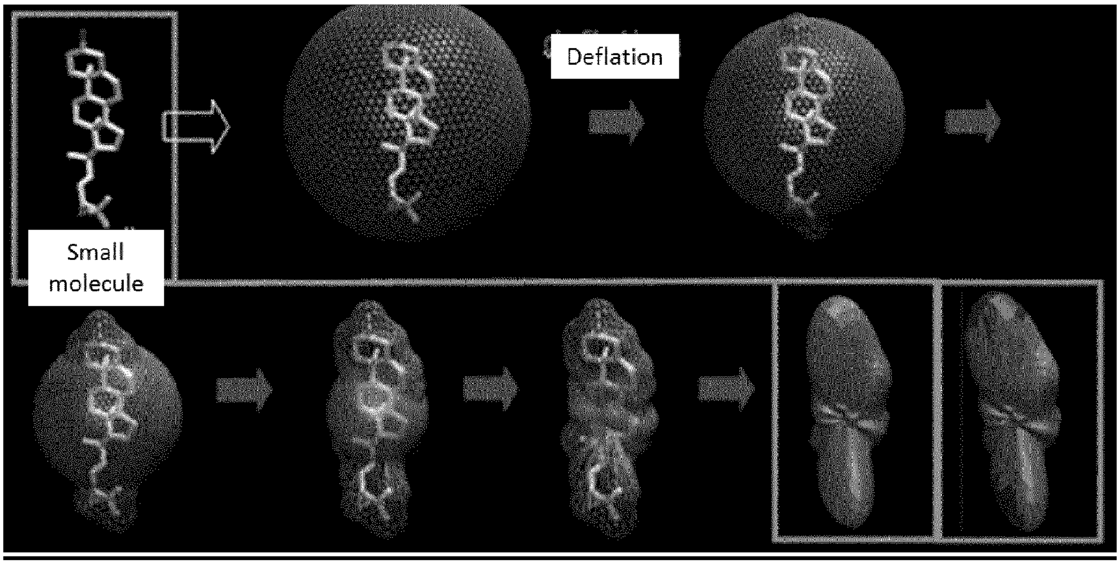


FIG. 1

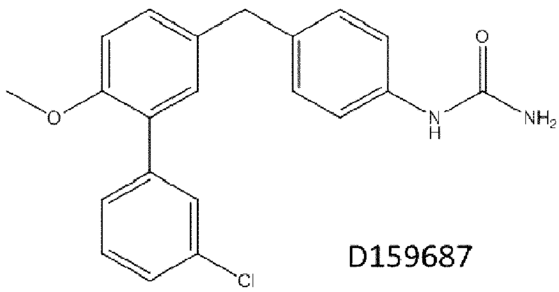

D159687

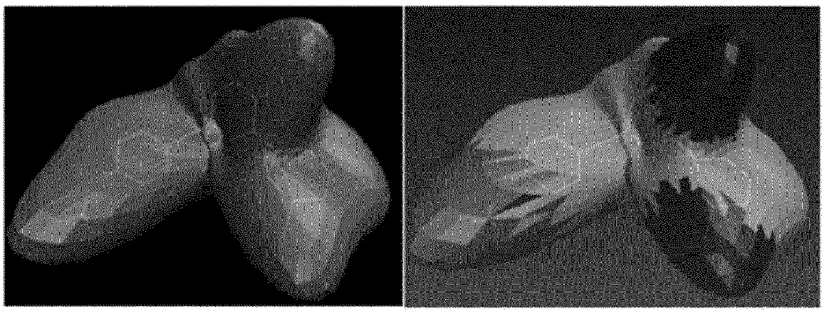

FIG. 2

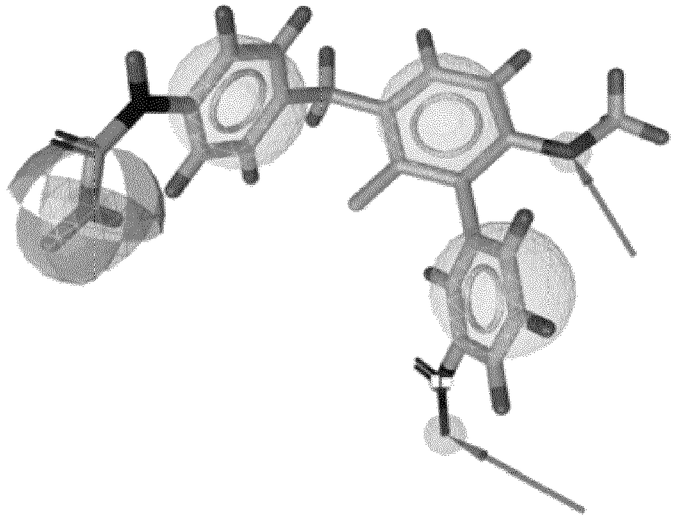

FIG. 3

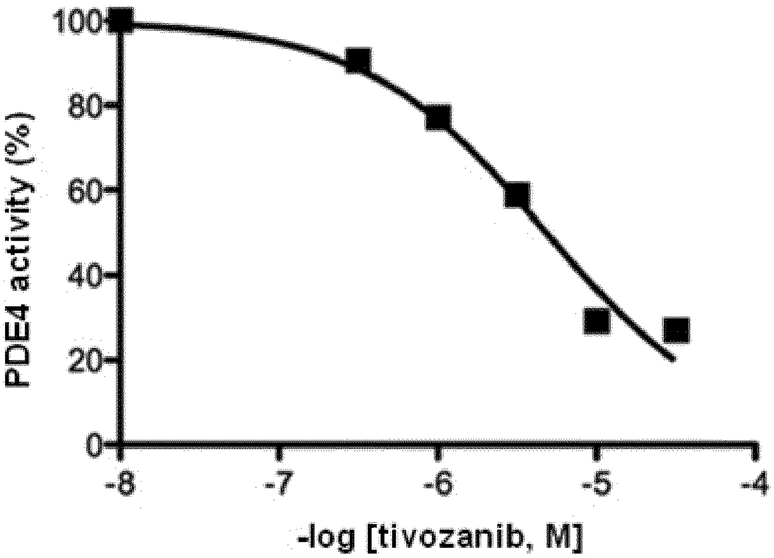


FIG. 4

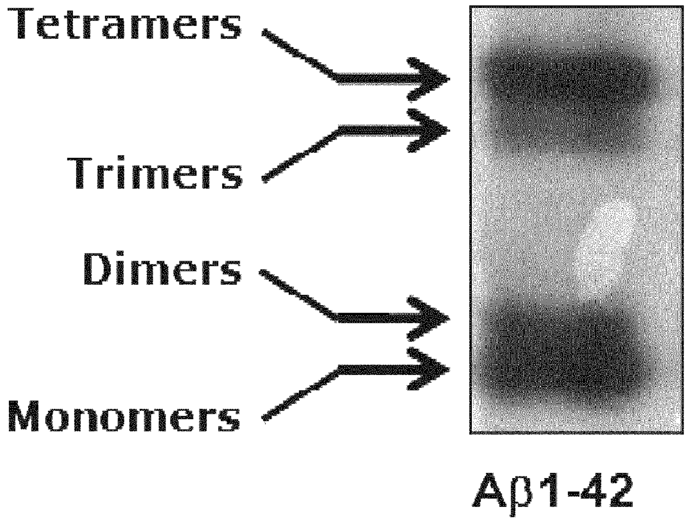
Tetramers
Trimers
Dimers
Monomers
Aβ1-42

FIG. 5

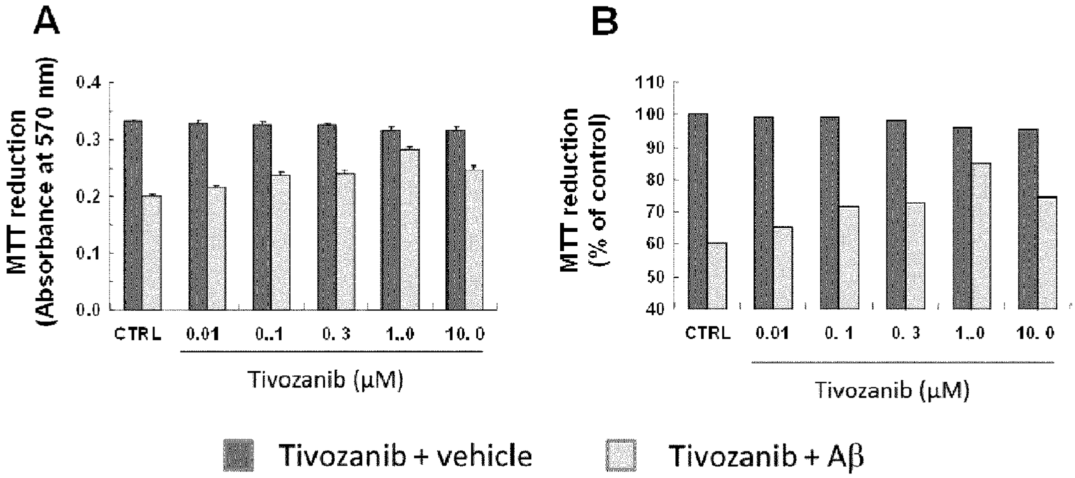
A
MTT reduction (Absorbance at 570 nm)
0.4
0.3
0.2
0.1
0.0
CTRL
0.01
0..1
0. 3
1..0
10. 0
Tivozanib (μM)
B
MTT reduction (% of control)
110
100
90
80
70
60
50
40
CTRL
0.01
0. 1
0. 3
1..0
10. 0
Tivozanib (μM)
Tivozanib + vehicle
Tivozanib + Aβ

FIG 6

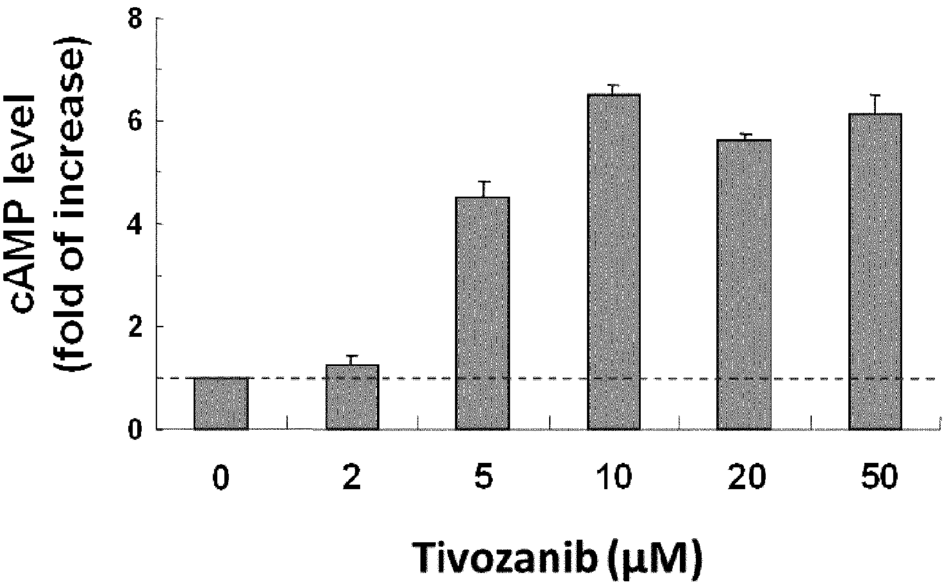
cAMP level
(fold of increase)
0
2
4
6
8
0
2
5
10
20
50
Tivozanib (µM)

FIG.7

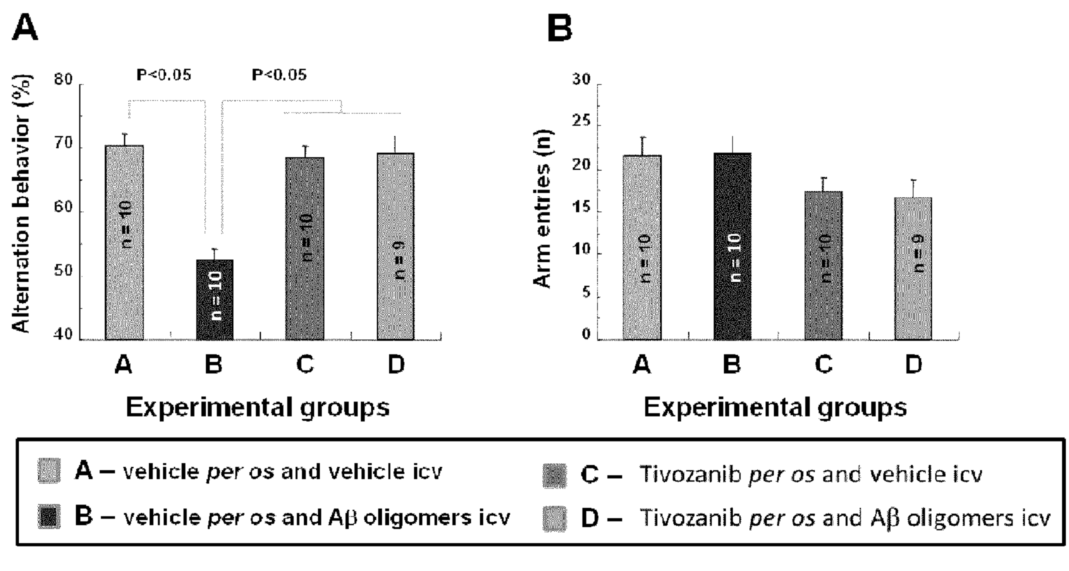
A
Alternation behavior (%)
P<0.05
P<0.05
n = 10
n = 10
n = 10
n = 9
A
B
C
D
Experimental groups
B
Arm entries (n)
n = 10
n = 10
n = 10
n = 9
A
B
C
D
Experimental groups
A – vehicle per os and vehicle icv
B – vehicle per os and Aβ oligomers icv
C – Tivozanib per os and vehicle icv
D – Tivozanib per os and Aβ oligomers icv

FIG 8

Europäisches Patentamt
European Patent Office
Office européen des brevets

# EUROPEAN SEARCH REPORT

Application Number
EP 12 18 1152

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 382 604 A1 (KIRIN BREWERY [JP]) 21 January 2004 (2004-01-21) * The claimed compounds as angiogenesis inhibitors and their use for the treatment of tumors, and Kaposis's sarcoma (a cancer type), atherosclerosis (a cardiovascular disease), chronic rheumatism and psoriasis (inflammatory conditions); see paragraphs [0070, 0071, 82, examples and claims 1, 29, 33) * ----- | 1-13 | INV. A61K31/4709 A61P25/28 A61P9/10 A61P17/06 A61P29/00 |
| X | EP 1 153 920 A1 (KIRIN BREWERY [JP]) 14 November 2001 (2001-11-14) * The claimed compounds, as angiogenesis inhibitors and their use for the treatment of tumors, and Kaposis's sarcoma (a cancer type), atherosclerosis (a cardiovascular disease), chronic rheumatism and psoriasis (inflammatory conditions); see paragraphs [0005, 0006, 0072, 0073, 0076, 0077], examples and claims. * ----- | 1-13 | |
| | | | TECHNICAL FIELDS SEARCHED (IPC) |
| X | EP 1 535 910 A1 (KIRIN BREWERY [JP]) 1 June 2005 (2005-06-01) * The claimed compounds, as inhibitors of macrophage colony.stimulated factor receptor autophosphorylation and their use for the treatment of cancer, and rheumatoid arthritis see paragraphs [0003, 0004], examples and claims 1, 90-92. According to the present application (see page 20) these conditions are "diseases involving the activation of phosphodiesterase 4". * ----- -/-- | 1-13 | A61K |

The present search report has been drawn up for all claims

| Place of search | Date of completion of the search | Examiner |
|---|---|---|
| Munich | 8 November 2012 | Veronese, Andrea |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 18 1152

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | UEMURA YASUNORI ET AL: "The selective M-CSF receptor tyrosine kinase inhibitor Ki20227 suppresses experimental autoimmune encephalomyelitis.", JOURNAL OF NEUROIMMUNOLOGY MAR 2008 LNKD- PUBMED:18378004, vol. 195, no. 1-2, March 2008 (2008-03), pages 73-80, XP002686634, ISSN: 0165-5728 * Ki20227, a compound falling under the definition of claim 1 has beneficial effects in animals affected by autoimmune encephalitis, i.e. a neurodegenerative disease. The compound is also considered a candidate to treat multiple sclerosis. See abstract, pg 74 (Ki20227), page 77, sections 3.4 and 3.5, conclusions, in particular page 79. * ----- | 1-14 | |
| Y | WO 2007/136225 A1 (IND ACADEMIC COOP [KR]; KWON YOUNG GUEN [KR]; MIN JEONG KI [KR]; YUN C) 29 November 2007 (2007-11-29) * Angiogenesis inhibitors for the treatment of Alzheimer's disease and also arthritis, psoriasis, atherosclerosis: see paragraphs [0013, 0018, 0020, 0023] and claims 1, 10 * ----- | 1-14 | TECHNICAL FIELDS SEARCHED (IPC) |

-/--

The present search report has been drawn up for all claims

| Place of search | Date of completion of the search | Examiner |
|---|---|---|
| Munich | 8 November 2012 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 12 18 1152

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | VAGNUCCI A H ET AL: "Alzheimer's disease and angiogenesis", THE LANCET, LANCET LIMITED. LONDON, GB, vol. 361, no. 9357, 15 February 2003 (2003-02-15), pages 605-608, XP004778573, ISSN: 0140-6736, DOI: 10.1016/S0140-6736(03)12521-4 * Antiangiogenesis inhibitors are suggested as potential candidates for treating alzheimer's disease: see abstract, figures and "testing the hypotesis" at page 607 * * See abstract, * ----- | 1-14 | |
| Y | DE FILIPPIS DANIELE ET AL: "Are anti-angiogenic drugs useful in neurodegenerative disorders?", CNS & NEUROLOGICAL DISORDERS, BENTHAM SCIENCE PUBLISHERS LTD, NL, vol. 9, no. 6, 1 December 2010 (2010-12-01), pages 807-812, XP009164397, ISSN: 1871-5273 * Antiangiogenesis agents and their use in the treatment of neurodegenerative diseases: Alzheimer's disease, Parkinson's disease and Multiple sclerosis (see abstract, and pages 809-810) * ----- -/-- | 1-14 | TECHNICAL FIELDS SEARCHED (IPC) |

The present search report has been drawn up for all claims

2

| Place of search | Date of completion of the search | Examiner |
|---|---|---|
| Munich | 8 November 2012 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 18 1152

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ESKENS FERRY A L M ET AL: "Biologic and clinical activity of tivozanib (AV-951, KRN-951), a selective inhibitor of VEGF receptor-1, -2, and -3 tyrosine kinases, in a 4-week-on, 2-week-off schedule in patients with advanced solid tumors.", CLINICAL CANCER RESEARCH : AN OFFICIAL JOURNAL OF THE AMERICAN ASSOCIATION FOR CANCER RESEARCH 15 NOV 2011 LNKD- PUBMED:21976547, vol. 17, no. 22, 15 November 2011 (2011-11-15), pages 7156-7163, XP002686635, ISSN: 1078-0432 * the whole document * ----- | 1-14 | |
| Y | PAGÈS LLUÍS ET AL: "PDE4 inhibitors: a review of current developments (2005 - 2009).", EXPERT OPINION ON THERAPEUTIC PATENTS NOV 2009 LNKD- PUBMED:19832118, vol. 19, no. 11, November 2009 (2009-11), pages 1501-1519, XP002686636, ISSN: 1744-7674 * the whole document * ----- | 1-14 | TECHNICAL FIELDS SEARCHED (IPC) |

The present search report has been drawn up for all claims

| Place of search | Date of completion of the search | Examiner |
|---|---|---|
| Munich | 8 November 2012 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 12 18 1152

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2012

| Patent document cited in search report | Publication date | Patent family member(s) | Publication date |
|---|---|---|---|
| EP 1382604 A1 | 21-01-2004 | AT 396988 T | 15-06-2008 |
| | | BR 0209216 A | 06-07-2004 |
| | | CA 2445333 A1 | 07-11-2002 |
| | | CN 1543459 A | 03-11-2004 |
| | | DE 60208364 T2 | 28-09-2006 |
| | | DK 1382604 T3 | 18-04-2006 |
| | | EP 1382604 A1 | 21-01-2004 |
| | | EP 1652847 A1 | 03-05-2006 |
| | | ES 2256466 T3 | 16-07-2006 |
| | | HK 1070649 A1 | 16-02-2007 |
| | | JP 2010077131 A | 08-04-2010 |
| | | MX PA03009662 A | 06-12-2004 |
| | | NO 20034595 A | 19-12-2003 |
| | | NZ 529046 A | 28-10-2005 |
| | | PL 367105 A1 | 21-02-2005 |
| | | RU 2283841 C2 | 20-09-2006 |
| | | TW I324154 B | 01-05-2010 |
| | | US 2003087907 A1 | 08-05-2003 |
| | | US 2004229876 A1 | 18-11-2004 |
| | | WO 02088110 A1 | 07-11-2002 |
| | | ZA 200307861 A | 08-10-2004 |
| EP 1153920 A1 | 14-11-2001 | AT 253051 T | 15-11-2003 |
| | | AT 356117 T | 15-03-2007 |
| | | AU 771504 B2 | 25-03-2004 |
| | | AU 3074800 A | 07-08-2000 |
| | | BR 0007656 A | 30-10-2001 |
| | | CA 2361057 A1 | 27-07-2000 |
| | | CN 1344254 A | 10-04-2002 |
| | | DE 60006216 D1 | 04-12-2003 |
| | | DE 60006216 T2 | 15-07-2004 |
| | | DE 60033857 T2 | 25-10-2007 |
| | | EP 1153920 A1 | 14-11-2001 |
| | | EP 1384712 A1 | 28-01-2004 |
| | | ES 2208261 T3 | 16-06-2004 |
| | | ES 2281591 T3 | 01-10-2007 |
| | | HK 1043792 A1 | 30-06-2005 |
| | | HU 0105133 A2 | 29-07-2002 |
| | | IL 144461 A | 04-07-2007 |
| | | JP 3519368 B2 | 12-04-2004 |
| | | NO 20012617 A | 14-09-2001 |
| | | NZ 513006 A | 31-10-2003 |
| | | PL 206627 B1 | 30-09-2010 |
| | | PL 349036 A1 | 01-07-2002 |
| | | TR 200102090 T2 | 21-01-2002 |
| | | TW I229667 B | 21-03-2005 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 12 18 1152

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2012

| Patent document cited in search report | Publication date | Patent family member(s) | Publication date |
|---|---|---|---|
| | | US 6797823 B1 | 28-09-2004 |
| | | US 2004209905 A1 | 21-10-2004 |
| | | US 2007027318 A1 | 01-02-2007 |
| | | WO 0043366 A1 | 27-07-2000 |
| EP 1535910 A1 | 01-06-2005 | AU 2003235838 A1 | 17-11-2003 |
| | | EP 1535910 A1 | 01-06-2005 |
| | | JP 2010168387 A | 05-08-2010 |
| | | US 2006235033 A1 | 19-10-2006 |
| | | WO 03093238 A1 | 13-11-2003 |
| WO 2007136225 A1 | 29-11-2007 | KR 20070113499 A | 29-11-2007 |
| | | WO 2007136225 A1 | 29-11-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1382604 A
- EP 1447405 A
- EP 1652847 A
- EP 1384712 A
- EP 1535910 A
- EP 0860433 A
- EP 1153920 A
- EP 1566379 A


### Non-patent literature cited in the description


- **BURGIN et al.** *Nature Biotechnology,* January 2010, vol. 28 (1), 63-72
- **GARCIA et al.** *The Journal of Neuroscience,* 02 June 2010, vol. 30 (22), 7516-7527
- **DESBENE et al.** *Neurobiology of Aging,* 2012, vol. 33, 1123.e17-1123.e29
- **YOUSSEF et al.** *Neurobiology of Aging,* 2008, vol. 29, 1319-1333